# EUROPEAN PATENT SPECIFICATION

(11) **EP 3 540 881 B1**
(45) Date of publication and mention of the grant of the patent: **17.06.2020**
(21) Application number: 19161102.9
(22) Date of filing: 06.03.2019
(51) Int. Cl.: H01T 23/00, B03C 3/08, B03C 3/12, A61L 9/22

(54) **PARTICLE COLLECTING DEVICE AND IMAGE FORMING APPARATUS**
PARTIKELSAMMELVORRICHTUNG UND BILDERZEUGUNGSVORRICHTUNG
DISPOSITIF DE COLLECTE DE PARTICULES ET APPAREIL DE FORMATION D'IMAGES

(30) Priority: 12.03.2018 JP 2018043759
(43) Date of publication of application: 18.09.2019
(73) Proprietor: Ricoh Company, Ltd., Tokyo 143-8555 (JP)
(72) Inventor: ISHIKAWA, Taisuke, Tokyo 143-8555 (JP)
(74) Representative: J A Kemp LLP

(56) References cited:
- EP-A1- 3 276 430
- WO-A1-2013/161534
- US-A1- 2010 201 776

## Description

### BACKGROUND

### Technical Field

The present invention relates to a particle collecting device that collects particles in the air, and an image forming apparatus equipped with such a particle collecting device.

### Discussion of the Background Art

In recent years, in the field of office equipment such as image forming apparatuses, a tendency to suppress a discharge amount of particles such as volatile organic compounds (VOC) and ultrafine particles (UFP) is increasing in order to suppress an influence of on the environment.

Here, as a particle collecting device that collects particles in the air, a device that ionizes particles and collects the particles with electrodes is known (see, for example, JP-5774212-B (WO2013/161534)). Such a particle collecting device is mounted in an exhaust path of the air for cooling in office equipment, thereby suppressing the discharge amount of the particles.

An image forming device with a particle collecting device is also disclosed in EP 3 276 430 A1.

Here, the office equipment requires a constant exhaust amount. Therefore, in mounting the particle collecting device in the exhaust path or the like, a pressure loss in the particle collecting device is desirably suppressed as much as possible. Meanwhile, the particle collecting device is required to improve the collection efficiency of the particles. However, structural considerations for improving the collection efficiency increase the pressure loss in some cases. As described above, there is still some room for improvement in the particle collecting devices in terms of suppression of the pressure loss and improvement of the collection efficiency of the particles.

### SUMMARY

In view of the above disadvantage, an object of the present invention is to provide a particle collecting device capable of achieving both suppression of a pressure loss and improvement of collection efficiency of particles.

Advantageously, the particle collecting device includes a duct, a blower, an ionizing device, at least two electrodes, a power supply, and a controller. The blower causes air to flow in a predetermined blowing direction inside the duct. The ionizing device is disposed inside the duct and ionizes particles contained in the air inside the duct. The at least two electrodes is disposed to extend along the blowing direction and faces each other on a downstream side of the ionizing device in the blowing direction inside the duct. The power supply charges at least one of the two electrodes to cause a potential difference between the two electrodes. The controller stops the blower in response to an ionization operation by the ionizing device.

Advantageously, an image forming apparatus includes a housing; an image forming device disposed inside the housing and configured to form an image on a recording medium; and the particle collecting device described above, disposed inside the housing.

According to an aspect of the present disclosure, since at least two electrodes facing each other extend along the blowing direction, a pressure loss can be suppressed. According to another aspect of the present disclosure, since the blower is stopped when the ionizing device performs the ionization operation, ionization can be performed in a state where the air including the particles is retained near the ionizing device. With the ionization, the ionization efficiency can be improved and the collection efficiency of the particles at the electrodes can be improved. Accordingly, both the suppression of the pressure loss and the improvement of the collection efficiency of the particles can be achieved.

### BRIEF DESCRIPTION OF THE DRAWINGS

The aforementioned and other aspects, features, and advantages of the present disclosure would be better understood by reference to the following detailed description when considered in connection with the accompanying drawings, wherein:
FIG. 1 is a diagram illustrating a schematic configuration of an image forming apparatus common to a first embodiment and a second embodiment;
FIG. 2 is a schematic diagram illustrating a particle collecting device of the first embodiment;
FIG. 3 is a schematic diagram illustrating a particle collecting device of the second embodiment;
FIG. 4 is a partially enlarged view illustrating that an electrode illustrated in FIG. 3 is a mesh-like member; and
FIG. 5 is a diagram illustrating a comparative example for comparison with the particle collecting devices of the first and second embodiments.

The accompanying drawings are intended to depict embodiments of the present disclosure and should not be interpreted to limit the scope thereof. The accompanying drawings are not to be considered as drawn to scale unless explicitly noted.

### DETAILED DESCRIPTION

In describing embodiments illustrated in the drawings, specific terminology is employed for the sake of clarity. However, the disclosure of this patent specification is not intended to be limited to the specific terminology so selected and it is to be understood that each specific element includes all technical equivalents that operate in a similar manner and achieve similar results.

Although the embodiments are described with technical limitations with reference to the attached drawings, such description is not intended to limit the scope of the disclosure and all of the components or elements described in the embodiments of this disclosure are not necessarily indispensable.

Referring now to the drawings, embodiments of the present disclosure are described below. In the drawings for explaining the following embodiments, the same reference codes are allocated to elements (members or components) having the same function or shape and redundant descriptions thereof are omitted below.

Embodiments of the present disclosure will be described below. As used herein, the singular forms "a", "an", and "the" are intended to include the plural forms as well, unless the context clearly indicates otherwise.

First, a schematic configuration of an image forming apparatus common to a first embodiment and a second embodiment to be described below will be described.

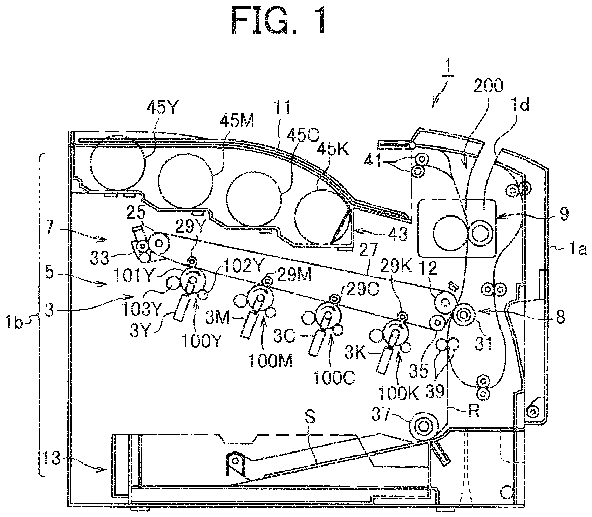

FIG. 1 is a diagram illustrating a schematic configuration of an image forming apparatus common to the first embodiment and the second embodiment.

An image forming apparatus 1 illustrated in FIG. 1 is used as a copying machine, a printer, a facsimile machine, or a multifunction peripheral of the aforementioned machines, which forms and fixes a toner image on a sheet-like recording medium S. In the present embodiment, a tandem-system full-color image forming apparatus will be described as an example of the image forming apparatus, but a four-cycle full-color image forming apparatus or the like can be used. Alternatively, the image forming apparatus can be a monochrome image forming apparatus or the like.

The image forming apparatus 1 includes a housing 1a, an image forming device 1b that is disposed inside the housing 1a, and a particle collecting device 200. The image forming device 1b forms an image on the recording medium S. The particle collecting device 200 is disposed inside the housing 1a. The particle collecting device 200 collects particles contained in the air inside the housing 1a and discharges the air to the outside of the housing 1a.

The image forming device 1b includes an exposure device 3 that emits laser light, and a process unit 5. In the process unit 5, an electrostatic latent image is formed with the laser light, and the process unit 5 forms a toner image from the electrostatic latent image. The image forming device 1b further includes an intermediate transfer unit 7 to which the toner image is transferred from the process unit 5, and a secondary transferer 8 that transfers the toner image from the intermediate transfer unit 7 to the recording medium S. Then, the image forming device 1b includes a fixing device 9 that fixes the toner image transferred by the secondary transferer 8 to the recording medium S.

The image forming device 1b further includes a stock tray 11 that temporarily holds the recording medium S to which the toner image is fixed, a sheet feeding tray 13 that accommodates a plurality of the recording media S, a discharge roller 41, and a bottle container 43 that accommodates a toner bottle. The discharge roller 41 discharges the recording medium S after fixation to the stock tray 11.

The exposure device 3 emits laser light according to image information of each of yellow, magenta, cyan, and black (hereinafter, "Y", "M", "C", "K" are attached to members corresponding to the respective colors). The exposure device 3 includes exposure units 3Y, 3M, 3C, and 3K of the respective colors. The exposure device 3 (that is, the exposure units 3Y, 3M, 3C, and 3K of the colors) is disposed below the process unit 5, as illustrated in FIG. 1, and irradiates the process unit 5 with the laser light upward from below.

The process unit 5 includes process cartridges 100Y, 100M, 100C, and 100K for forming respective toner images of yellow, magenta, cyan, and black. Each of the process cartridges 100Y, 100M, 100C, and 100K is attachable to and detachable from the housing 1a of the image forming apparatus 1 so that the process cartridges 100Y, 100M, 100C, and 100K can be replaced at the expiration of life or at the consumption of the cartridge. Meanwhile, the exposure units 3Y, 3M, 3C, and 3K that emit the laser light toward the process cartridges 100Y, 100M, 100C, and 100K are secured to the housing 1a of the image forming apparatus 1.

The process cartridges 100Y, 100M, 100C, and 100K have similar configurations to one another except that the colors of the toners to be used are different. Hereinafter, the configuration of the yellow process cartridge 100Y will be described as a representative example. Further, in addition, the exposure unit will be described by taking the yellow exposure unit 3Y as an example.

As illustrated in FIG. 1, the process cartridge 100Y includes a photoconductor drum 101Y, a charger 102Y, and a developing device 103Y. The photoconductor drum 101Y is a drum-shaped photoconductor that rotates. The charger 102Y charges an outer peripheral surface of the photoconductor drum 101Y. Then, the exposure unit 3Y is disposed on a downstream side of the charger 102Y in a rotating direction of the photoconductor drum 101Y.

In the present embodiment, the exposure unit 3Y is disposed below the photoconductor drum 101Y, and emits the laser light upward toward the outer peripheral surface of the photoconductor drum 101Y from below. The charged outer peripheral surface of the photoconductor drum 101Y is exposed with the laser light. By the exposure, an electrostatic latent image is formed on the outer peripheral surface of the photoconductor drum 101Y.

The developing device 103Y is disposed on a downstream side of the exposure unit 3Y in the rotating direction of the photoconductor drum 101Y, and develops the electrostatic latent image formed by the exposure. The toner image formed by the development is transferred to the intermediate transfer unit 7 at a transfer position on a downstream side of the developing device 103Y in the rotating direction of the photoconductor drum 101Y, as will be described below.

The intermediate transfer unit 7 transfers the toner images from the process cartridges 100Y, 100M, 100C, and 100K to the surface of the recording medium S. The intermediate transfer unit 7 includes an endless intermediate transfer belt 27, and a cleaning backup roller 25 and a secondary transfer backup roller 12, around which the intermediate transfer belt 27 is stretched, and which allow the intermediate transfer belt 27 to circle. The intermediate transfer unit 7 further includes primary transfer rollers 29Y, 29M, 29C, and 29K of the respective colors disposed across the intermediate transfer belt 27 between the intermediate transfer unit 7 and the photoconductor drums of the process cartridges 100Y, 100M, 100C, and 100K. The intermediate transfer unit 7 further includes a belt cleaning device 33. The intermediate transfer belt 27 circles counterclockwise on FIG. 1 in a state of being held by predetermined tension by a tension roller 35.

The primary transfer rollers 29Y, 29M, 29C, and 29K are are applied with a bias having a polarity opposite a polarity the toner charge. With this applied bias, the toner is attracted from the surface of the photoconductor drum of each of the process cartridges 100Y, 100M, 100C, and 100K toward the intermediate transfer belt 27 to primarily transfer the toner image to the intermediate transfer belt 27.

The secondary transferer 8 includes a secondary transfer roller 31 that presses the recording medium S against the intermediate transfer belt 27. The secondary transfer roller 31 is provided to face the secondary transfer backup roller 12. The secondary transfer roller 31 is applied with a bias having a polarity opposite to the polarity of the toner charge, and the toner is attracted from the surface of the intermediate transfer belt 27 toward the recording medium S to secondarily transfer the toner image to the recording medium S.

The belt cleaning device 33 includes a cleaning blade made of an elastic body such as urethane rubber that abuts on the intermediate transfer belt 27. The cleaning blade comes in contact with the intermediate transfer belt 27 to block and remove a transfer residual toner on the surface of the intermediate transfer belt 27.

The fixing device 9 receives the recording medium S on which the toner image has been secondarily transferred in a nip formed by two rotators at least one of which carries heat. The fixing device 9 heats and pressurizes the recording medium S to fix the toner image on the recording medium S.

The sheet feeding tray 13 is provided in a lower portion of the housing 1a of the image forming apparatus 1. A plurality of the recording media S such as paper is accommodated in the sheet feeding tray 13. The recording media S are pulled out from the sheet feeding tray 13 one by one by a sheet feeding roller 37 and conveyed along a recording medium conveying path R formed inside the housing 1a.

The bottle container 43 is provided in an upper portion of the housing 1a. Toner bottles 45Y, 45M, 45C, and 45K are accommodated in the bottle container 43. The toner bottle 45Y is filled with a yellow toner corresponding to a color separation component of a color image. The toner bottle 45M is filled with a magenta toner corresponding to a color separation component of the color image. The toner bottle 45C is filled with a cyan toner corresponding to a color separation component of the color image. The toner bottle 45K is filled with a black toner corresponding to a color separation component of the color image.

The toners of the respective colors in the toner bottles 45Y, 45M, 45C, and 45K are appropriately supplied to the respective developing devices of the process cartridges 100Y, 100M, 100C, and 100K. The toner bottles 45Y, 45M, 45C, and 45K are attachable to and detachable from the housing 1a independently of the process cartridges 100Y, 100M, 100C, and 100K.

Here, in the present embodiment, the particle collecting device 200 is provided to take in the air heated in the above-described fixing device 9, at a constant flow rate, and discharges the air through an exhaust port 1d. The fixing device 9 is cooled by such an exhaust operation. At this time, particles such as VOC and UFP may be generated in the fixing device 9. The particle collecting device 200 takes in the particles together with the air, collects the particles from the air, and discharges the air after from which the particles are removed.

Next, a basic operation of the image forming apparatus 1 configured as described above will be described.

When an image forming operation is started in the image forming apparatus 1, the respective photoconductor drums of the process cartridges 100Y, 100M, 100C, and 100K are rotationally driven. Then, the surfaces of the photoconductor drums are charged.

Next, the charged surfaces of the photoconductor drums are exposed by the exposure units 3Y, 3M, 3C, and 3K, and electrostatic latent images are formed. At this time, image information used for the exposure to the photoconductor drums is monochrome image information obtained by decomposing a desired full-color image into color information of yellow, magenta, cyan, and black.

Next, the electrostatic latent images on the surfaces of the photoconductor drums are developed to form toner images of the respective colors. The toner images of the respective colors are sequentially superimposed and transferred by the primary transfer rollers 29Y, 29M, 29C, and 29K onto the intermediate transfer belt 27 circling counterclockwise in FIG. 1. At this time, a full-color toner image is carried on the intermediate transfer belt 27.

Further, the sheet feeding roller 37 is rotated by a driving device, and one recording medium S is sent out from the sheet feeding tray 13 to the recording medium conveying path R. Timing is controlled by a pair of registration rollers 39, and the recording medium S sent out to the recording medium conveying path R is sent to between the secondary transfer roller 31 and the secondary transfer backup roller 12 facing the secondary transfer roller 31. Then, the full-color toner image on the intermediate transfer belt 27 is transferred onto the recording medium S by the secondary transfer roller 31. Further, the transfer residual toner remaining on the surface of the intermediate transfer belt 27 after the transfer of the toner image is removed by the belt cleaning device 33.

Next, the recording medium S to which the toner image has been transferred is conveyed to the fixing device 9. In the fixing device 9, the recording medium S is received in the nip and nipped and conveyed. At that time, heat and pressure are applied to the recording medium S at the nip. With the application of heat and pressure, the toner image is fixed on the recording medium S. Then, the recording medium S having passed through the nip is discharged to the stock tray 11 by the discharge roller 41.

Further, at this time, the particle collecting device 200 takes in the air heated generated by the fixing. After collecting the particles such as VOC and UFP, the particle collecting device 200 discharges the air through the exhaust port 1d.

The image forming apparatus 1 performs such a basic operation to form a full-color image on the recording medium S. In addition to such an operation, a single-color image can be formed using at least one of the process cartridges 100Y, 100M, 100C, and 100K, or a two-color or three-color image can be formed using two or three of the process cartridges 100Y, 100M, 100C, and 100K.

Next, descriptions are given below of examples of the above-described particle collecting device 200 that can be mounted in the image forming apparatus 1. First, the particle collecting device of the first embodiment will be described.

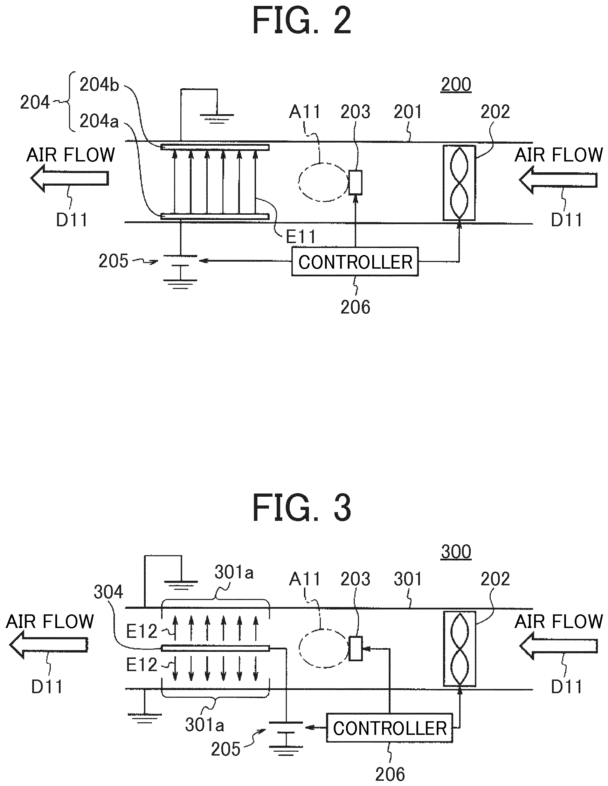

FIG. 2 is a schematic diagram illustrating a particle collecting device of the first embodiment.

The particle collecting device 200 illustrated in FIG. 2 includes a duct 201, a blower 202, an ionizing device 203, two electrodes 204, a power supply 205, and a controller 206. The controller 206 can be a processor including a central processing unit (CPU) and associated memory units such as a read only memory (ROM), a random access memory (RAM), etc. The processor performs various types of control processing by executing programs stored in the memory.

The duct 201 is a tube formed of an insulating material extending from the inside of the fixing device 9 illustrated in FIG. 1 to the exhaust port 1d provided in the housing 1a of the image forming apparatus 1.

The blower 202 is a fan that causes the air to flow in the duct 201 in a blowing direction D11 from the inside of the fixing device 9 to the exhaust port 1d.

The ionizing device 203 is a device disposed inside the duct 201, to ionize the particles contained in the air inside the duct 201. In the ionizing device 203, as a built-in high-voltage power supply applies a high voltage to electrodes, the electrodes perform corona discharge. With the corona discharge, the ionizing device 203 ionizes the particles in the air in a neighboring area A11.

The two electrodes 204 are disposed on a downstream side of the ionizing device 203 in the blowing direction D11 inside the duct 201 . The two electrodes 204 are face each other and extend along the blowing direction D11. One of the two electrodes 204 is a charging electrode 204a and the other is an earth electrode 204b. The charging electrode 204a is an electrode to which a voltage is applied by the power supply 205, and the earth electrode 204b is an electrode connected to ground in the housing 1a of the image forming apparatus 1. In the present embodiment, both of the charging electrode 204a and the earth electrode 204b are shaped like flat metal plates, and are disposed adjacent to or on an inner face of the duct 201.

The power supply 205 is a power supply that charges the charging electrode 204a to cause a potential difference between the two electrodes 204. An electric field E11 from the charging electrode 204a toward the earth electrode 204b occurs due to the potential difference caused by the charging.

The controller 206 is configured to control operations of the blower 202, the ionizing device 203, and the power supply 205. The controller 206 is configured to stop the blower 202 when the ionizing device 203 performs the ionization operation.

In the particle collecting device 200 having the above configuration, first, the blower 202 causes the air to flow in the blowing direction D11 under the control of the controller 206. With the flow of the air, the air containing the particles generated in the fixing device 9 is taken into the duct 201. At a point of time when the air has been taken in to some extent, the controller 206 stops the blower 202 and causes the ionizing device 203 to perform the ionization operation. Since the blower 202 stops, the ionizing device 203 ionizes the particles in the air retained in the neighboring area A11. After having the ionizing device 203 perform the ionization operation for a certain time, the controller 206 causes the blower 202 to resume blowing and causes the power supply 205 to apply the voltage to the charging electrode 204a. Along the electric field E11 that occurs at this time, the particles ionized by the ionizing device 203 move toward the charging electrode 204a and the earth electrode 204b and are collected by the charging electrode 204a and the earth electrode 204b. The air from which the particles are removed is discharged as is through the exhaust port 1d. Hereinafter, the particle collecting device 200 repeatedly executes the above series of operations at predetermined timing while the image forming apparatus 1 is powered on.

Next, the particle collecting device of the second embodiment will be described.

FIG. 3 is a schematic diagram illustrating a particle collecting device of the second embodiment. In FIG. 3, constituent elements equivalent to the constituent elements in FIG. 2 are denoted by the same reference signs as FIG. 2, and redundant description of the equivalent constituent elements is hereinafter omitted.

First, a particle collecting device 300 illustrated in FIG. 3 includes a blower 202, an ionizing device 203, a power supply 205, and a controller 206 as constituent elements equivalent to the constituent elements of the particle collecting device 200 of the first embodiment illustrated in FIG. 2. Meanwhile, the particle collecting device 300 includes a duct 301 and a single electrode 304 as constituent elements different from the constituent elements of the particle collecting device 200 of the first embodiment.

The duct 301 is a tube extending from an inside of the fixing device 9 illustrated in FIG. 1 to the exhaust port 1d provided in the housing 1a of the image forming apparatus 1. In the present embodiment, the entire wall face including a partial wall face 301a facing the electrode 304 is a metal tube formed of a conductive metal material. Then, the wall face of the duct 301, that is, the partial wall face 301a is connected to ground in the housing 1a of the image forming apparatus 1. That is, in the particle collecting device 300 of the second embodiment, the partial wall face 301a facing the electrode 304 serves as an earth electrode in the duct 301.

The ionizing device 203 is disposed on an upstream side of the partial wall face 301a in a blowing direction D11, that is, on an upstream side of the electrode 304 facing the partial wall face 301a, inside the duct 301.

The single electrode 304 is disposed inside the duct 301 to extend along the blowing direction D11 and to face the partial wall face 301a. In the present embodiment, the single electrode 304 serves as a charging electrode to which a voltage is applied from the power supply 205. A potential difference is caused between the partial wall face 301a and the electrode 304 due to the charging. With the potential difference, an electric field E12 occurs from each of both surfaces of the electrode 304 toward the partial wall face 301a facing the each of the surfaces. Here, in the present embodiment, the electrode 304 is a mesh-like member as follows.

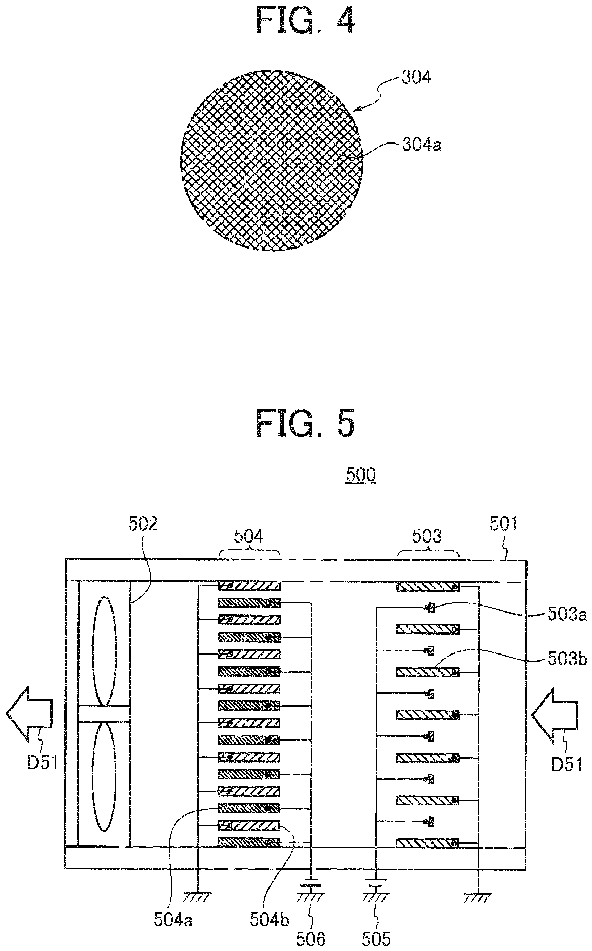

FIG. 4 is a partially enlarged view illustrating that the electrode illustrated in FIG. 3 is a mesh-like member.

As illustrated in FIG. 4, the electrode 304 is a metal mesh knitted with a plurality of metal wires 304a. As a result, the surface area of the electrode 304 is substantially larger than a case where the electrode 304 is formed of a simple metal plate.

In the particle collecting device 300 of the second embodiment illustrated in FIG. 3, the blower 202 causes the air to flow in the blowing direction D11, and the air containing particles having occurred in the fixing device 9 is taken into the duct 201 under the control of the controller 206. Next, the controller 206 stops the blower 202 and causes the ionizing device 203 to perform an ionization operation. After having the ionizing device 203 perform the ionization operation for a certain time, the controller 206 causes the blower 202 to resume blowing and causes the power supply 205 to apply a voltage to the electrode 304. Along the electric field E12 that occurs at this time, the ionized particles move toward and are collected by the electrode 304 and the partial wall face 301a of the duct. The air after the particles are collected is discharged through the exhaust port 1d. The particle collecting device 300 repeatedly executes the above-described series of operations at predetermined timing.

FIG. 5 is a diagram illustrating a comparative example for comparison with the particle collecting devices of the first and second embodiments.

In a particle collecting device 500 of the comparative example illustrated in FIG. 5, the air is taken into a duct 501 by a blower 502 in a blowing direction D51, and particles contained in the air are ionized by an ionizing electrode 503 and are attracted and collected by a collecting electrode 504. The ionizing electrode 503 has a plurality of point-like charging electrodes 503a and flat plate-like earth electrodes 503b alternately arranged, and a voltage is applied from an ionization power supply 505 to each of the point-like charging electrodes 503a. Further, the collecting electrode 504 has a plurality of flat plate-like charging electrodes 504a and a plurality of flat plate-like earth electrodes 504b alternately arranged, and a voltage is applied from a collection power supply 506 to each of the plate-like charging electrodes 504a.

The particle collecting device 500 of this comparative example does not perform the above-described operation control like the particle collecting device 200 or 300 of the first or second embodiment. The blower 502 continues to cause the air to flow, and the ionization power supply 505 and the collection power supply 506 continue to apply the voltage on a constant basis during the operation of the particle collecting device 500 of the comparative example.

Here, in the particle collecting device 500 of the comparative example, the flat plate-like earth electrodes 503b and the point-like charging electrodes 503a are provided in a plurality of pairs, to improve the ionization efficiency in the ionizing electrode 503. The flat plate-like earth electrodes 503b are disposed to extend along the blowing direction D51. However, due to stacking in a louver manner of the plurality of the flat plate-like earth electrodes 503b, a pressure loss inevitably occurs in the air flowing through the flat plate-like earth electrodes 503b. The same applies to the collecting electrode 504.

According to the particle collecting device 200 or 300 of the first or second embodiment, the two particle collection electrodes 204 (or the electrode 304 and the partial partial wall face 301a)facing each other are disposed to extend along the blowing direction D11. Therefore, the pressure loss can be suppressed in the electrodes 204. Further, the blower 202 stops when the ionizing device 203 performs the ionization operation. Therefore, the ionization can be performed in the state where the air containing the particles is retained in the neighboring area A11 of the ionizing device 203. With the ionization, the ionization efficiency can be improved and the collection efficiency of the particles at the electrodes 204 or the electrode 304 can be improved. Furthermore, the pressure loss in the ionizing device can be suppressed by simplification of the ionizing device 203 in expectation of improvement of the ionization efficiency and reduction of the number of the particle collection electrodes 204 or 304.

First, as for the ionizing device 203, the single device is disposed near a center of a thickness direction of the duct 201 or 301 and is simplified in the particle collecting device 200 or 300 of the first or second embodiment. Although the single ionizing device 203 is not specified here, the ionizing device 203 may include a single pair of the flat plate-like earth electrode 503b and the point-like charging electrode 503a illustrated in FIG. 5, for example. With such simplification, an air flow space in the duct 201 or 301 can be widened to suppress the pressure loss around the ionizing device 203.

In the particle collecting device 200 of the first embodiment, the number of the electrodes 204 is reduced to two: the charging electrode 204a and the earth electrode 204b. Since the number of the electrodes 204 is two, the electrodes 204 are disposed near the inner surface of the duct 201 to extend along the blowing direction D11 and the air flow space is widened, whereby the pressure loss can be suppressed. Further, in the particle collecting device 300 of the second embodiment, the number of the electrodes 304 is reduced to one (the charging electrode 204a). Therefore, the air flow space in the duct 301 is widened and the pressure loss can be suppressed.

As described above, according to the particle collecting device 200 or 300 of the first or second embodiment, the pressure loss can be suppressed in both the ionizing device 203 and the electrodes 204 or the electrode 304, and the collection efficiency of the particles can be improved. That is, both the suppression of the pressure loss and the improvement of the collection efficiency of the particles can be achieved.

Further, in the particle collecting device 200 of the first embodiment, one of the two electrodes 204 is connected to ground. Since the grounded earth electrode 204b does not require charging, the power supply 205 is simplified. As described above, in the particle collecting device 200 of the first embodiment, the number of the electrodes is reduced to two and the charging is performed by the single charging electrode 204a. Therefore, the entire apparatus including the power supply 205 can be simplified and manufacturing cost can be reduced.

Further, according to the particle collecting device 200 of the first embodiment, the two electrodes 204 are the plate-like members, which are easily processed. Therefore, parts cost of the electrodes 204 can be reduced.

Further, in the particle collecting device 300 of the second embodiment, the partial wall face 301a facing the electrode 304 is connected to ground in the duct 301, and the power supply 205 charges the electrode 304 alone. With the configuration, the charging is performed by the electrode 304 alone, and thus the power supply 205 can be simplified and the manufacturing cost can be reduced.

Further, according to the particle collecting device 300 of the second embodiment, the electrode 304 is the mesh-like member and the surface area contributing to the collection of the ionized particles is large. Therefore, the collection efficiency of the particles can be further improved.

In the image forming apparatus 1 illustrated in FIG. 1, one of the particle collecting devices 200 and 300 according to the first and second embodiments is used to collect the particles contained in the air inside the housing 1a. With the configuration, both the suppression of the pressure loss and the improvement of the collection efficiency of the particles can be achieved for exhaust from the housing 1a.

Note that the above-described embodiments are merely representative forms of the present invention, and the present invention is not limited to these embodiments. That is, a person skilled in the art can make and implement various modifications within a range not deviating from the gist of the present invention according to conventionally known knowledge. Even such modifications are of course included in the scope of the present invention as long as the modifications have any of the configurations of the particle collecting device and the image forming apparatus of the present invention.

For example, in the above-described first and second embodiments, the electrophotographic image forming apparatus 1 has been exemplified as an example of the image forming apparatus. However, the image forming apparatus is not limited to the example and can be, for example, an inkjet image forming apparatus.

In the above-described first and second embodiments, the particle collecting devices 200, and 300 that collect the particles contained in the air of the fixing device 9 in the electrophotographic image forming apparatus 1 have been exemplified as examples of the particle collecting device. However, the particle collecting device is not limited to the examples and can be a device that collects particles contained in the air at another place in the image forming apparatus. Alternatively, the particle collecting device can be a device that collects particles contained in the air of equipment other than the image forming apparatus or can be a device installed alone in a room or the like and which collects particles contained in the ambient air.

Further, in the above-described first embodiment, the flat plate-like electrodes 204 have been exemplified as the two electrodes 204 facing each other in the particle collecting device 200 of the first embodiment, as an example of electrodes. Further, in the particle collecting device 300 of the second embodiment, the mesh-like electrode 304 has been exemplified as the single electrode 304 facing the partial wall face 301a of the duct 301. However, the electrodes are not limited to the example. For example, the electrodes facing each other can be mesh-like electrodes, and the electrode facing the partial wall face of the duct can be a flat plate like electrode. Alternatively, one of the electrodes facing each other can be a flat plate-like electrode and the other can be a mesh-like electrode.

Further, in the above-described first or second embodiment, the ionizing device 203 that is a single device disposed near the center in the thickness direction of the duct 201 or 301 has been exemplified as an example of the ionizing device. However, the ionizing device is not limited to the example, and the number of installations and installation positions can be arbitrarily set within an allowable range for the pressure loss in the particle collecting device.

Further, in the above-described first embodiment, the two electrodes 204 adjacent to the inner surface of the duct 201 have been exemplified as an example of "at least two electrodes". However, the "at least two electrodes" is not limited to the example, and the number of installations and installation positions can be arbitrarily set within an allowable range for the pressure loss in the particle collecting device.

Further, in the above-described first embodiment, the power supply 205 that charges the charging electrode 204a alone of the two electrodes 204 has been exemplified as an example of the power supply. Further, in the second embodiment, the power supply 205 that charges the electrode 304 alone, of the partial wall face 301a of the duct 301 and the electrode 304, has been exemplified. However, the power supply is not limited to the example. The power supply may charge all of electrodes to have potentials different from one another as long as the power supply charges at least two electrodes to have a potential difference. Further, the power supply may charge a partial wall face of a duct and an electrode facing the partial wall face to have potentials different from each other as long as the power supply charges the partial wall face and the electrode to have a potential difference.

Further, in the above-described first embodiment, the duct 301 has been exemplified as an example of "a duct having at least a partial wall face formed of a conductive material". The entire duct 301 including the partial wall face 301a facing the electrode 304 is formed of the conductive metal. However, the "duct having at least a partial wall face formed of a conductive material" is not limited to the example, and the partial wall face can be formed of a conductive material and the remaining wall face can be formed of an insulating material.

Further, in the above-described first embodiment, the particle collecting device 200 in which the two electrodes 204 of the charging electrode 204a and the earth electrode 204b are disposed inside the duct 201 has been exemplified as an example of the particle collecting device. Further, in the second embodiment, the particle collecting device 300 in which the single electrode 304 is disposed inside the duct 301, both surfaces of the electrode 304 facing the partial wall face 301a, has been exemplified. However, the particle collecting device is not limited to the examples, and these two embodiments can be combined, for example. That is, two electrodes facing, with one surface, a partial wall face of a duct can be disposed in parallel and away from each other, and one or more pairs of electrodes such as a charging electrode and an earth electrode can be disposed in parallel between the two electrodes.

## Claims

1. A particle collecting device (200) comprising:
a duct (201);
a blower (202) configured to cause air to flow in a predetermined blowing direction (D11) inside the duct (201);
an ionizing device (203) disposed inside the duct (201) and configured to ionize particles contained in the air inside the duct (201);
at least two electrodes (204; 301a,304) extending along the predetermined blowing direction (D11) and facing each other, the at least two electrodes (204; 301a,304) disposed downstream from the ionizing device (203) inside the duct (201) in the predetermined blowing direction (D11);
a power supply (205) configured to charge at least one of the at least two electrodes (204; 301a,304) to cause a potential difference between the at least two electrodes (204; 301a,304); **characterized by**
a controller (206) configured to:
control the blower (202), the ionizing device (203), and the power supply (205); and
stop the blower (202) in response to an ionization operation by the ionizing device (203).

2. The particle collecting device (200) according to claim 1,
wherein at least one of the at least two electrodes (204; 301a,304) is grounded.

3. The particle collecting device (200) according to claim 2,
wherein the at least two electrodes (204; 301a,304) are two electrodes (204; 301a,304), and
wherein one of the two electrodes (204; 301a,304) is grounded.

4. The particle collecting device (300) according to claim 1 or 2,
wherein the at least two electrodes (301a,304) are two electrodes (301a,304), and
wherein one of the two electrodes (301a,304) is a wall face (301a) of the duct (301) facing the other (304) of the two electrodes (301a,304), the wall face (301a) formed of a conductive material.

5. The particle collecting device (300) according to claim 4,
wherein the wall face (301a) of the duct (301) is grounded, and
the power supply (205) is configured to charge only the other (304) of the two electrode (301a,304).

6. The particle collecting device (200 or 300) according to any one of claims 1 to 5,
wherein the at least two electrodes (204; 301a,304) includes an electrode (204a; 204b) shaped into a flat plate.

7. The particle collecting device (200 or 300) according to any one of claims 1 to 5,
wherein the at least two electrodes (204; 301a,304) includes an electrode (304) shaped into a mesh.

8. An image forming apparatus (1) comprising:
a housing (1a);
an image forming device (1b) disposed inside the housing (1a) and configured to form an image on a recording medium (S); and
the particle collecting device (200 or 300) according to any one of claims 1 to 7, disposed inside the housing (1a),
wherein the particle collecting device (200 or 300) is configured to collect particles contained in air inside the housing (1a) and discharge the air to an outside of the housing (1a).

## Patentansprüche

1. Teilchensammelvorrichtung (200), umfassend:
einen Kanal (201);
ein Gebläse (202), das konfiguriert ist, um zu verursachen, dass Luft in eine vorbestimmte Blasrichtung (D11) in dem Kanal (201) strömt;
eine Ionisierungsvorrichtung (203), die in dem Kanal (201) angeordnet ist und konfiguriert ist, um Teilchen, die in der Luft in dem Kanal (201) enthalten sind, zu ionisieren;
mindestens zwei Elektroden (204; 301a, 304), die sich entlang der vorbestimmten Blasrichtung (D11) erstrecken und einander zugewandt sind, wobei die mindestens zwei Elektroden (204; 301a, 304) stromabwärts der Ionisierungsvorrichtung (203) in dem Kanal (201) in der vorbestimmten Blasrichtung (D11) angeordnet sind;
eine Energieversorgung (205), die konfiguriert ist, um mindestens eine der mindestens zwei Elektroden (204; 301a, 304) zu laden, um eine Potentialdifferenz zwischen den mindestens zwei Elektroden (204; 301a, 304) zu verursachen, **gekennzeichnet durch**
eine Steuerung (206), die für Folgendes konfiguriert ist:
Steuern des Gebläses (202), der Ionisierungsvorrichtung (203) und der Energieversorgung (205); und
Anhalten des Gebläses (202) als Reaktion auf einen Ionisierungsvorgang durch die Ionisierungsvorrichtung (203).

2. Teilchensammelvorrichtung (200) nach Anspruch 1,
wobei mindestens eine der mindestens zwei Elektroden (204; 301a, 304) geerdet ist.

3. Teilchensammelvorrichtung (200) nach Anspruch 2,
wobei die mindestens zwei Elektroden (204; 301a, 304) zwei Elektroden (204; 301a, 304) sind und
wobei eine der zwei Elektroden (204; 301a, 304) geerdet ist.

4. Teilchensammelvorrichtung (300) nach Anspruch 1 oder 2,
wobei die mindestens zwei Elektroden (301a, 304) zwei Elektroden (301a, 304) sind und
wobei eine der zwei Elektroden (301a, 304) eine Wandfläche (301a) des Kanals (301) ist, die der anderen (304) der zwei Elektroden (301a, 304) zugewandt ist, wobei die Wandfläche (301a) aus einem leitfähigen Material gebildet ist.

5. Teilchensammelvorrichtung (300) nach Anspruch 4,
wobei die Wandfläche (301a) des Kanals (301) geerdet ist und
die Energieversorgung (205) konfiguriert ist, um die andere (304) der zwei Elektroden (301a, 304) zu laden.

6. Teilchensammelvorrichtung (200 oder 300) nach einem der Ansprüche 1 bis 5,
wobei die mindestens zwei Elektroden (204; 301a, 304) eine Elektrode (204a; 204b) beinhalten, die in eine flache Platte geformt ist.

7. Teilchensammelvorrichtung (200 oder 300) nach einem der Ansprüche 1 bis 5,
wobei die mindestens zwei Elektroden (204; 301a, 304) eine Elektrode (304) beinhalten, die in ein Gitter geformt ist.

8. Bilderzeugungsgerät (1), umfassend:
ein Gehäuse (1a);
eine Bilderzeugungsvorrichtung (1b), die in dem Gehäuse (1a) angeordnet ist und konfiguriert ist, um ein Bild auf einem Aufzeichnungsmedium (S) zu erzeugen; und
die Teilchensammelvorrichtung (200 oder 300) nach einem der Ansprüche 1 bis 7, die in dem Gehäuse (1a) angeordnet ist,
wobei die Teilchensammelvorrichtung (200 oder 300) konfiguriert ist, um Teilchen zu sammeln, die in Luft in dem Gehäuse (1a) enthalten sind, und um die Luft an ein Äußeres des Gehäuses (1a) abzugeben.

## Revendications

1. Dispositif collecteur de particules (200) comprenant :
un conduit (201) ;
une soufflante (202) configurée pour faire circuler l'air dans une direction de soufflage prédéterminée (D11) à l'intérieur du conduit (201) ;
un dispositif ionisant (203) disposé à l'intérieur du conduit (201) et configuré pour ioniser les particules contenues dans l'air à l'intérieur du conduit (201) ;
au moins deux électrodes (204 ; 301a, 304) s'étendant le long de la direction de soufflage prédéterminée (D11) et se faisant face, les au moins deux électrodes (204 ; 301a, 304) étant disposées en aval du dispositif d'ionisation (203) à l'intérieur du conduit (201) dans la direction de soufflage prédéterminée (D11) ;
une alimentation (205) configurée pour charger au moins l'une des au moins deux électrodes (204 ; 301a, 304) pour provoquer une différence de potentiel entre les au moins deux électrodes (204 ; 301a, 304) ; **caractérisé par**
un contrôleur (206) configuré pour :
contrôler la soufflante (202), le dispositif d'ionisation (203) et l'alimentation électrique (205) ; et
arrêter la soufflante (202) en réponse à une opération d'ionisation par le dispositif d'ionisation (203).

2. Dispositif collecteur de particules (200) selon la revendication 1, dans lequel au moins une des au moins deux électrodes (204 ; 301a, 304) est mise à la terre.

3. Dispositif collecteur de particules (200) selon la revendication 2,
dans lequel les au moins deux électrodes (204 ; 301a, 304) sont deux électrodes (204 ; 301a, 304), et dans lequel l'une des deux électrodes (204; 301a, 304) est mise à la terre.

4. Dispositif collecteur de particules (300) selon la revendication 1 ou 2,
dans lequel les au moins deux électrodes (301a, 304) sont deux électrodes (301a, 304), et
dans lequel l'une des deux électrodes (301a, 304) est une face de paroi (301a) du conduit (301) face à l'autre (304) des deux électrodes (301a, 304), la face de paroi (301a) étant formée d'un matériau conducteur.

5. Dispositif collecteur de particules (300) selon la revendication 4,
dans lequel la face de paroi (301a) du conduit (301) est mise à la terre, et
l'alimentation électrique (205) est configurée pour charger uniquement l'autre (304) des deux électrodes (301a, 304).

6. Dispositif collecteur de particules (200 ou 300) selon l'une quelconque des revendications 1 à 5,
dans lequel les au moins deux électrodes (204 ; 301a, 304) incluent une électrode (204a ; 204b) conformée en une plaque plate.

7. Dispositif collecteur de particules (200 ou 300) selon l'une quelconque des revendications 1 à 5,
dans lequel les au moins deux électrodes (204; 301a, 304) incluent une électrode (304) conformée en un maillage.

8. Appareil de formation d'images (1) comprenant :
un boîtier (1a) ;
un dispositif de formation d'image (1b) disposé à l'intérieur du boîtier (1a) et configuré pour former une image sur un support d'enregistrement (S) ; et
le dispositif collecteur de particules (200 ou 300) selon l'une quelconque des revendications 1 à 7, disposé à l'intérieur du boîtier (la),
dans lequel le dispositif collecteur de particules (200 ou 300) est configuré pour collecter les particules contenues dans l'air à l'intérieur du boîtier (1a) et évacuer l'air vers l'extérieur du boîtier (1a).
